(19) Europäisches Patentamt
European Patent Office
Office européen des brevets

(11) Veröffentlichungsnummer: **0 345 658 A2**

# (12) EUROPÄISCHE PATENTANMELDUNG

(21) Anmeldenummer: **89110004.2**

(22) Anmeldetag: **02.06.89**

(51) Int. Cl.4: **C12N 11/08 , C12P 41/00 , //C12N9/20**

(30) Priorität: **08.06.88 DE 3819467**

(43) Veröffentlichungstag der Anmeldung:
**13.12.89 Patentblatt 89/50**

(84) Benannte Vertragsstaaten:
**DE ES FR GB IT NL SE**

(71) Anmelder: **BASF Aktiengesellschaft**
**Carl-Bosch-Strasse 38**
**D-6700 Ludwigshafen(DE)**

(72) Erfinder: **Urban, Dieter, Dr.**
**Espenstrasse 15**
**D-6800 Mannheim 1(DE)**
Erfinder: **Ladner, Wolfgang, Dr.**
**In den Bellen 5**
**D-6701 Fussgoenheim(DE)**
Erfinder: **Paul, Axel, Dr.**
**Kaefertaler Strasse 38**
**D-6800 Mannheim 1(DE)**
Erfinder: **Sanner, Axel, Dr.**
**Lorscher Ring 2c**
**D-6710 Frankenthal(DE)**

## (54) Verfahren zur Herstellung eines Biokatalysators und dessen Verwendung zur Razematspaltung.

(57) Verfahren zur Herstellung eines Biokatalysators durch Mischen von

a) hydrophilierten Polyolefinfasern,

b) roher Schweinepankreaslipase,

c) mindestens einem wasserlöslichen zwei- bis sechswertigen Alkohol mit 2 bis 6 Kohlenstoffatomen und

d) einer wäßrigen Pufferlösung vom pH 5 bis 9

bei 0 bis 40 °C, nach 1 Minute bis 3 Tagen Filtrieren und Waschen des Filterkuchens,
sowie die Verwendung des so erhaltenen Biokatalysators zur Racematspaltung von Estern racemischer Alkohole in wasserhaltigem Medium.

EP 0 345 658 A2

## Verfahren zur Herstellung eines Biokatalysators und dessen Verwendung zur Razematspaltung

Die Erfindung betrifft ein einfaches und wirtschaftliches Verfahren zur Herstellung eines Immobilisates von roher Pankreaslipase auf Basis einer hydrophilierten Polyolefinfaser, das Immobilisat selbst und dessen Verwendung zur Razematspaltung von Estern razemischer Alkohole.

Techniken zur Immobilisierung von Lipase sind bekannt. Gemäß US 4 629 742 wird zur Immobilisierung Lipase aus Candida cylindracea eingesetzt, und als Träger hydrophobe, mikroporöse Polymere, die in einem sehr aufwendigen Verfahren hergestellt wurden.

Das in der EP-A-210 499 u.a. auch für Pankreaslipase beschriebene Immobilisierungsverfahren durch Gelbildung aus Enzym, Polyepoxid und Polyamin ist zwar einfach, führt jedoch zu Biokatalysatoren, die infolge ihrer geringen mechanischen Stabilität und schlechter hydrodynamischer Eigenschaften (Filtrierbarkeit; Strömungswiderstand in damit gepackten Säulen) für den technischen Gebrauch wenig geeignet sind.

Auch die enantioselektive Spaltung razemischer Ester durch Schweinepankreaslipase im Labormaßstab ist bekannt (W.E. Ladner und G.M. Whitesides, JACS 1984, 106, 7250 - 7251). Dabei wurde rohe, nicht immobilisierte Lipase eingesetzt. Dieses Verfahren ist zur technischen Durchführung ungeeignet, ein wirtschaftliches Verfahren erfordert eine Immobilisierung. Eine solche nach üblichen Methoden, etwa nach dem obengenannten US-Patent, führte nicht zum Ziel, denn die spezifische Aktivität des Immobilisats war niedrig und ließ im Betrieb viel zu rasch nach.

Der Erfindung lag daher die Aufgabe zugrunde, Immobilisate roher Pankreaslipase leicht und preiswert herzustellen, so daß ein einfaches, wirtschaftliches und für den technischen Maßstab geeignetes Verfahren zur Razematspaltung von Estern optisch aktiver Alkohole durchführbar ist. Wichtige Kriterien eines geeigneten Biokatalysators sind lange Lebensdauer und hohe Raum-Zeit-Ausbeuten.

Die Lösung der Aufgabe besteht in dem Verfahren nach den Ansprüchen 1 bis 9. Die Herstellung des Biokatalysators erfolgt durch Mischen einer hydrophylierten Polyolefinfaser, einer Lipase und Wasser bei pH 5 bis 9, vorzugsweise 5,5 bis 8 und 0 bis 40, vorzugsweise 10 bis 30 °C, nach einer Minute bis 3 Tagen, vorzugsweise ein bis 24 Stunden, Filtrieren und Waschen des Filterkuchens mit Wasser oder Pufferlösung oder einem wasserlöslichen organischen Lösungsmittel, wobei als Lipase rohe, d.h. bis zu 99 Gew.% natürliche Verunreinigungen (vor allem Proteine) enthaltende und damit sehr leicht und preiswert zugängliche, handelsübliche Schweinepankreaslipase eingesetzt wird, und wobei der wäßrigen Mischung als Stabilisator für die Lipase (zur zeitlichen Verlängerung ihrer Aktivität) mindestens ein wasserlöslicher aliphatischer Alkohol mit zwei bis sechs Kohlenstoffatomen und zwei bis sechs Hydroxylgruppen, gegebenenfalls im Gemisch mit einem einwertigen Alkohol mit 1 bis 4 Kohlenstoffatomen, zugesetzt wird.

Mit Polyolefinen sind Polyethylen, Polypropylen und deren Mischungen und Mischpolymerisate gemeint.

Das Hydrophilieren (zwecks Verbesserung der Wasserbenetzbarkeit) kann nach einer der folgenden Methoden erfolgen:

1. Die fertige Faser kann mit einem Hydrophilierungsmittel behandelt werden, beispielsweise mit Polyvinylalkohol (nach DE-PS 22 37 606) oder mit einem Melamin-Formaldehyd-Polykondensat (nach DE-PS 23 46 081 oder 25 45 727).
2. Bei der Herstellung der Fasern, z.B. nach DE-AS 21 17 370, kann man der Polyolefinlösung das Hydrophilierungsmittel (beispielsweise eines der oben genannten) zusetzen, so daß dieses mechanisch in die Fasern eingebaut wird (vgl. z.B. DE-OS 22 08 553).
3. Copolymerisieren des die Faser bildenden Olefins mit hydrophilen Comonomeren wie Vinylacetat (mit anschließender Verseifung), N-Vinylformamid, N-Butylacrylat, Acrylsäure, Acrylamid, (vgl. z.B. US 4,210,615.

Besonders bewährt hat sich Polyethylen-Synthese-Pulp der Firma Schwarzwälder Textilwerke, D-7623 Schenkenzell.

Die mittlere Länge der Polyolefinfasern soll entsprechend dem jeweiligen Einsatz im Bereich von 0,05 bis 10, vorzugsweise 0,1 bis 4 mm liegen. Die Untergrenze wird durch die Filtrierbarkeit bzw. den hydrodynamischen Widerstand einer damit gepackten Säule bestimmt. Das Verhältnis von Länge zu Durchmesser beträgt mindestens 5:1 und liegt im allgemeinen im Bereich von 10:1 bis 20:1. Die Menge der zur besseren Benetzung mit Wasser eingesetzten Hydrophilierungsadditive beträgt 0,1 bis 10, vorzugsweise 0,4 bis 4 %, bezogen auf das Fasergewicht.

Die Lipase selbst ist zwar wasserlöslich, doch sind viele Bestandteile der rohen Lipase wasserunlöslich und werden im Reaktionsgemisch suspendiert.

Mit wasserlöslichen Alkoholen sind solche gemeint, die bei 20 °C zu mindestens 10, besser zu mindestens 20 Gew.% in Wasser löslich sind. Besonders bevorzugt sind Alkohole, die in jedem Verhältnis mit Wasser mischbar sind. Mit der "Wertigkeit" des Alkohols ist die Zahl der Hydroxylgruppen gemeint. Als wasserlösliche Alkohole kömmen beispielsweise in Betracht:

Ethylenglykol, 1,2- und 1,3-Propandiol, 1,2-, 1,3-, 1,4- und 2,3-Butandiol, 1,2- und 1,3-i-Butandiol, 1,2- und 1,5-Pentandiol, Diethylenglykol, Glyzerin, Erythrit, Pentaerythrit, Arabit, Xylit, Sorbit, Mannit und Dulcit und deren Mischungen. Ein Teil dieser mehrwertigen Alkohole kann auch durch einen oder mehrere folgender einwertiger Alkohole ersetzt sein: Methanol, Ethanol, 1- und 2-Propanol, 2-Butanol und t-Butanol. Bevorzugt werden mehrwertige wasserlösliche Alkohole mit 2 bis 6 OH-Gruppen allein oder in Mischungen verschiedenwertiger Alkohole, insbesondere Glyzerin und Mischungen aus Glyzerin mit Methanol, Ethanol, 1,5-Pentandiol und insbesondere 2-Propanol.

Zur Einstellung des pH-Wertes im Bereich von 5 bis 9, vorzugsweise 5,5 bis 8, verwendet man zweckmäßig die üblichen Puffermischungen, beispielsweise Mischungen von primären und sekundären Phosphaten, Borax, Mischungen von Tris-(hydroxymethyl)-methylamin und dessen Hydrochlorid, Acetatpuffermischungen.

Die Gesamtmischung setzt sich folgendermaßen zusammen:

1 bis 50, vorzugsweise 2 bis 30 % hydrophilierte Polyolefinfasern,

1 bis 30, vorzugsweise 5 bis 25 % rohe Schweinepankreaslipase,

1 bis 68, vorzugsweise 20 bis 60 % mindestens eines der beschriebenen wasserlöslichen, mehrwertigen Alkohole, wobei die über 1, vorzugsweise über 5, insbesondere 10 % hinausgehende Menge durch einen wasserlöslichen einwertigen Alkohol mit 1 bis 4 C-Atomen ersetzt sein kann,

30 bis 97 % Wasser

sowie den pH-Wert regulierende Zusätze (die gewichtsmäßig kaum eine Rolle spielen). Die Prozente beziehen sich jeweils auf das Gewicht der Gesamtmischung.

Die Komponenten können in beliebiger Reihenfolge gemischt werden, vorzugsweise suspendiert man die rohe Lipase in der Mischung aus Wasser und Alkohol und gibt danach die Fasern zu.

Die Mischung läßt man zweckmäßig eine Minute bis 3 Tage, vorzugsweise 1 Stunde bis 1 Tag bei 0 bis 40, vorzugsweise 10 bis 30 °C verweilen, wobei man sie vorzugsweise in Bewegung hält (z. B. Rühren, Rollen). Dann wird filtriert. Der Filterkuchen kann unmittelbar, gewaschen oder ungewaschen, in wasserhaltigem Medium zur Esterspaltung eingesetzt werden. Vorteilhaft wird er aber vorher mit einer wäßrigen oder wäßrig-alkoholischen Lösung eines Vernetzungsmittels zur Fixierung der Lipase eine Minute bis 2 Stunden, vorzugsweise 2 Minuten bis 1 Stunde lang bei Raumtemperatur (10 bis 30 °C) behandelt, d.h. in Kontakt gebracht. Geeignete Vernetzungsmittel sind z.B. Dialdehyde, insbesondere Glutardialdehyd und Glyoxal, sowie wasserlösliche Glycidylether, hergestellt gemäß EP-A 210 499, durch Anlagerung von 1 bis 30 Äquivalenten eines Epoxids der Formel I

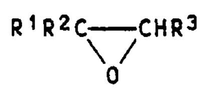

I,

worin die Reste $R^1$, $R^2$ und $R^3$ Wasserstoffatome oder Methyl- oder Ethylgruppen darstellen, an je 1 Äquivalent (bezogen auf OH-Gruppen) eines polyfunktionellen Alkohols mit 2 bis 6 Kohlenstoffatomen oder eines Mono-oder Disaccharids und Umsetzung des so erhaltenen Anlagerungsproduktes mit pro OH-Gruppe einem Äquivalent Epichlorhydrin und anschließende Cyclisierung zum Epoxid.

Eine Vernetzung beeinträchtigt zwar die Anfangsaktivität etwas, weshalb nicht zu stark vernetzt werden sollte, doch bleibt die Aktivität bei wiederholtem Einsatz relativ stabil (Beispiele 1, 2 und 5).

Die Verwendung des Immobilisates kann in üblicher Weise, z.B. in Säulen oder im Rührreaktor, erfolgen.

Durch das erfindungsgemäße Verfahren wird zum ersten Mal Schweinepankreaslipase in ein wiederverwendbares, gut filtrierbares, für den Einsatz in Säulen geeignetes Immobilisat von hoher spezifischer Aktivität und langer Einsatzdauer überführt. Die Tatsache, daß Nebenbestandteile roher Lipase dabei nicht stören, macht das Verfahren besonders wirtschaftlich.

Bestimmung der enzymatischen Aktivität von Pankreatin

In einer pH-Stat-Apparatur werden 50 ml Borax/HCl-Puffer (20 mM, pH 7,5) und 1 ml Glycidylbutyrat

miteinander gemischt und bei 30 °C intensiv gerührt. Nach Zugabe einer geeigneten Menge (üblicherweise 1-10 mg) roher Pankreaslipase bzw. des zu testenden Immobilisats (500 mg) wird durch automatische Dosierung von 0,5 N NaOH-Lösung der pH konstant gehalten. Die spezifische enzymatische Aktivität $A_{sp}$ wird wie folgt berechnet:

$$A_{sp} = \frac{\Delta V \cdot N}{\Delta t \cdot m} .$$

Dabei bedeuten:
$\Delta V \Delta t$ den Verbrauch der Natronlauge zu Beginn der Reaktion in ml/min, N die Normalität der Natronlauge (500 µmol/ml) und m die Einwaage des Enzyms bzw. des Immobilisates.

Die übliche Einheit für die enzymatische Aktivität ist 1 unit (Abkürzung: u), das entspricht einem NaOH-Verbrauch von 1 µmol/min.

Die spezifische Aktivität des zur Immobilisierung verwendeten Pankreatins der Fa. Nordmark betrug 17 u/mg.

Die in den Beispielen genannten Teile und Prozente beziehen sich auf das Gewicht. Zur Esterspaltung wurde stets Buttersäure-Glycidyl-Ester eingesetzt.

Herstellung des Immobilisates

Beispiel 1

In 18 g einer Mischung aus 80 Teilen 200 mmolarer Phosphatpufferlösung vom pH 7,0, 10 Teilen Glyzerin und 10 Teilen Isopropanol suspendierte man 2 g rohe Schweinepankreaslipase. Zu dieser Suspension gab man 1 g Polyethylen- Synthesepulp SWP ESS 21F der Firma Schwarzwälder Textilwerke Schenkenzell, bestehend aus 0,5 g Polyethylenfasern (mittlere Faserlänge 0,6 mm, Polyvinylalkoholgehalt 4 %) und 0,5 g Wasser. Die Mischung wurde über Nacht gerührt und anschließend mit 5,4 g Glutardialdehyd (25%ig in Wasser) versetzt. Nach einer weiteren Stunde Rühren wurde abfiltriert und mit einer Mischung aus 80 Teilen 200 mmolarer Phosphatpufferlösung vom pH 7,0, 10 Teilen Glyzerin und 10 Teilen Isopropanol gewaschen und gut abgesaugt. Man erhielt 2,0 g Immobilisat. Dieses wurde fünfmal zur Esterspaltung eingesetzt, die spezifischen Enzymaktivitäten stehen in der Tabelle:

| Ansatz | $A_{sp}$ [u/g] | $A_{rel}$ (in %) |
|---|---|---|
| 1 | 1449 | - |
| 2 | 1307 | 90 |
| 3 | 1212 | 84 |
| 4 | 1111 | 77 |
| 5 | 1026 | 71 |
| $A_{sp}$ = spezifische Aktivität in<br>$A_{rel}$ = relative Aktivität, bezogen auf die ursprüngliche | | |

Beispiel 2

Wie Beispiel 1, aber ohne Vernetzung mit Glutardialdehyd. Man erhielt 2,4 g Immobilisat.

| Ansatz | $A_{sp}$ [u/g] | $A_{rel}$ (in %) |
|---|---|---|
| 1 | 3700 | - |
| 2 | 2381 | 64 |
| 3 | 1498 | 40 |
| 4 | 1149 | 31 |
| 5 | 828 | 22 |

Beispiel 3

Wie Beispiel 1, jedoch mit der Hälfte Schweinepankreaslipase. Man erhielt 1,9 g Immobilisat.

| Ansatz | $A_{sp}$ [u/g] | $A_{rel}$ (in %) |
|---|---|---|
| 1 | 1333 | - |
| 2 | 1093 | 82 |
| 3 | 950 | 71 |
| 4 | 910 | 68 |
| 5 | 901 | 68 |

Beispiel 4

Wie Beispiel 1, jedoch nur 1/4 Schweinepankreaslipase. Man erhielt 1,7 g Immobilisat.

| Ansatz | $A_{sp}$ [u/g] | $A_{rel}$ (in %) |
|---|---|---|
| 1 | 606 | - |
| 2 | 513 | 85 |
| 3 | 476 | 79 |
| 4 | 427 | 70 |
| 5 | 416 | 69 |

Beispiel 5

Wie Beispiel 1, jedoch mit der Hälfte Glutardialdehyd. Man erhielt 2,1 g Immobilisat.

| Ansatz | $A_{sp}$ [u/g] | $A_{rel}$ (in %) |
|---|---|---|
| 1 | 2516 | - |
| 2 | 2469 | 98 |
| 3 | 2381 | 95 |
| 4 | 2150 | 85 |
| 5 | 2083 | 83 |

Beispiel 6

In 18 g einer Mischung aus 65 Teilen 50 mmolaren Boratpufferlösung vom pH 8,0, 30 Teilen Glyzerin und 5 Teilen Ethanol suspendierte man 1 g rohe Schweinepankreaslipase. Zu dieser Suspension gab man 1 g Polyethylen-Syn thesepulp SWP E-780 der bei Beispiel 1 genannten Firma, bestehend aus 0,35 g Polyethylenfasern (mittlere Faserlänge 1,6 mm, Polyvinylalkoholgehalt 0,4 % und 0,65 g Wasser. Die Mischung wurde 3 Stunden gerührt, dann mit 2,5 g Sorbit-$EO_{80}$-Epoxid (s.unten) versetzt und eine weitere Stunde gerührt. Das Immobilisat wurde abgesaugt und mit obiger Mischung aus Boratpuffer, Glyzerin und Ethanol gewaschen. Man erhielt 2,6 g Immobilisat. Dieses wurde dreimal zur Esterspaltung eingesetzt, die spezifischen Aktivitäten sind in der Tabelle aufgeführt.

| Ansatz | $A_{sp}$ [u/g] | $A_{rel}$ (in %) |
|---|---|---|
| 1 | 1010 | - |
| 2 | 960 | 95 |
| 3 | 814 | 81 |

Das Sorbit-$EO_{80}$-Epoxid hatte einen Epoxidwert von 1,2 mmol/g und wurde wie folgt hergestellt (DE 35 27 014):
3705 g Sorbit-$EO_{80}$ (Reaktionsprodukt aus 1 Mol Sorbit mit 80 Mol Ethylenoxid, vgl. Houben-Weyl, Methoden der Org. Chemie 14/2, (1963), S. 450) werden mit 14,8 g $BF_3$-Dihydrat versetzt. Danach werden bei 70 °C unter Rühren 555 g Epichlorhydrin zugetropft. Es wird weitere 2 Stunden bei 70 °C gerührt und anschließend bei 20 bis 35 °C 528 g 50 %ige Natronlauge im Verlauf von 1 bis 2 Stunden zugetropft. Es wird so lange weitergerührt, bis etwa 90 % der Natronlauge verbraucht sind. Der Natronlauge-Verbrauch wird titrimetrisch verfolgt.

Die Hauptmenge des Wassers wird bei 70 °C im Wasserstrahlvakuum abdestilliert und der Rest heiß (70 °C) abgesaugt.

So werden 3439 g (85 %) Umsetzungsprodukt von Epichlorhydrin mit Sorbit-$EO_{80}$ erhalten, welches einen Epoxid-Titer von 1,2 mmol/g besitzt.

Beispiel 7

In 18 g einer Mischung aus 200 mmolarer Phosphatpufferlösung vom pH 8,0 und Alkohol (s. Tabelle) suspendierte man 2 g rohe Schweinepankreaslipase. Zu dieser Suspension gab man 1 g Polyethylensynthesepulp SWP E-790 der o.g. Firma, bestehend aus 0,5 g Polyethylenfasern (mittlere Faserlänge 1,6 mm, Polyvinylalkoholgehalt 0,7 %) und 0,5 g Wasser. Diese Mischung wurde über Nacht gerührt, mit 2,7 g Glutardialdehyd (25%ig in Wasser) versetzt und eine Stunde gerührt. Das Immobilist wurde abgesaugt und mit der jeweiligen Alkohol-Phosphatpuffer-Lösung gewaschen.

| Alkohol [Gew.-Teile] | | Phosphatpuffer [Gew.-Teile] | Ansatz | $A_{sp}$ [u/g] | $A_{rel.}$ in % |
|---|---|---|---|---|---|
| - | Glyzerin 12 | 88 | 1 | 833 | - |
| | | | 2 | 833 | 100 |
| Isopropanol 10 | Glyzerin 20 | 80 | 1 | 2469 | - |
| | | | 2 | 2083 | 84 |
| 1,5-Pentandiol 10 | Glyzerin 10 | 80 | 1 | 1546 | - |
| | | | 2 | 1380 | 89 |
| Methanol 10 | Glyzerin 10 | 80 | 1 | 1050 | - |
| | | | 2 | 940 | 90 |

Beispiel 8

In 540 g einer Mischung aus 80 Teilen 200 mmolarer Phosphatpufferlösung vom pH 7,0, 10 Teilen Glyzerin und 10 Teilen Isopropanol suspendierte man 60 g rohe Schweinepankreaslipase. Zu dieser Suspension gab man 30 g Polyethylen-Synthesepulp SWP ESS 21F der o.g. Firma, bestehend aus 15 g Polyethylenfasern (mittlere Faserlänge 0,6 mm, Polyvinylalkoholgehalt 4 %) und 15 g Wasser. Die Mischung wurde über Nacht gerollt und anschließend mit 81 g Glutardialdehyd (25%ig in Wasser) versetzt. Nach einer weiteren Stunde Rühren wurde abfiltriert und mit 810 g der o.g. Mischung aus 80 Teilen Phosphatpufferlösung, 10 Teilen Glyzerin und 10 Teilen Isopropanol gewaschen und gut abgesaugt. Man erhielt 82 g Immobilisat mit einer spez. Aktivität bei 30 °C von 2469 u/g. Dieses Immobilisat wurde im Anwendungsbeispiel verwendet.

Vergleichsversuch 1 (mit einem anderen Träger)

2 g rohe Schweinepankreaslipase wurden gemäß EP-A-210 499 in 8 g einer Lösung aus 50 Teilen 50 mmolarem Phosphatpuffer $p_H$ 8,0 und 50 Teilen Glyzerin suspendiert und mit 6,5 g Sorbit-$EO_{80}$-epoxid (siehe Beispiel 6) und 4,0 g Polyethyleniminlösung (25 % im Wasser, mit HCl auf $p_H$ 8,0 eingestellt) gemischt. Nach 24 Stunden wurde das entstandene Gel durch ein Sieb mit 1 mm Maschenbreite gedrückt.

| Ansatz | $A_{sp}$ [u/g] | $A_{rel}$ (in %) |
|---|---|---|
| 1 | 53 | 100 |
| 2 | 32 | 60 |
| 3 | 28 | 53 |

Vergleichsversuch 2 (mit einem anderen Träger)

In 18 g einer Mischung aus 80 Teilen 200 mmolarer Phosphatpufferlösung vom pH 8,0, 10 Teilen Glyzerin und 10 Teilen Isopropanol suspendierte man 2 g rohe Schweinepankreaslipase. Zu dieser Suspension gab man 1 g poröses Polyethylen, hergestellt nach US-PS 4 247 498 (Accurel® HDPE-Pulver, Enka AG, Wuppertal) und ließ über Nacht rühren. Eine Stunde nach der Zugabe von 5,4 g Glutardialdehyd (25 %ig in Wasser) wurde abgesaugt. Man erhielt 2,1 g Immobilisat.

| Ansatz | $A_{sp}$ [u/g] | $A_{rel.}$ in % |
|---|---|---|
| 1 | 120 | 100 |
| 2 | 73 | 61 |

Vergleichsversuch 3

Wie Beispiel 5, jedoch wurde der Alkohol durch Phosphatpufferlösung vom pH 8,0 ersetzt.

| Ansatz | $A_{sp}$ [u/g] | $A_{rel}$ (in %) |
|---|---|---|
| 1 | 470 | 100 |
| 2 | 439 | 93 |
| 3 | 388 | 83 |
| 4 | 384 | 82 |
| 5 | 328 | 70 |

Vergleichsversuch 4

Analog zu Beispiel 1 wurden vier andere Lipasen eingesetzt:
- Lipase Saiken 100 aus Rhizopus japonicus von K.K. Osaka Saihin Kenkyusho
- Lipase M-AP 10 aus Mucor sp. von Amano Pharmaceutical Co. Ltd.
- Piccantase-A aus Mucor miehei von Gist-Brocades
- Lipase My aus Candida cylindracea von Meito Sangyo

Die ersten drei zeigten keine enzymatische Aktivität bei der Esterspaltung, die spezifischen Aktivitäten der Lipase My stehen in der Tabelle:

| Ansatz | $A_{sp}$ [u/g] | $A_{rel.}$ in % |
|---|---|---|
| 1 | 399 | - |
| 2 | 280 | 70 |

Anwendungsbeispiel

1,4 l einer 0,05 molaren, auf pH 7 eingestellten Pufferlösung ($Na_2B_4O_7.10H_2$, mit konzentrierter Salzsäure eingestellt) wurden auf 6° C gekühlt. Es wurden 216,1 g (1,5 Mol) razemisches Glycidylbutyrat und 10 g feuchtes, mit pH-7-Pufferlösung gewaschenes Immobilisat aus Beispiel 8 zugegeben. Das Gemisch wurde bei 9 bis 10° C intensiv gerührt und durch Zupumpen von 10 N NaOH (insgesamt 82,5 ml, 0,825 Mol, entsprechend 55 % Hydrolyse) nahe pH 7,3 gehalten. Nach 130 Min. war die angegebene NaOH-Menge verbraucht. Das Immobilisat wurde abfiltriert, zweimal mit $CH_2Cl_2$, einmal mit pH-8-Puffer gewaschen und anschließend, in pH-7-Puffer suspendiert, bei 5 bis 8° C aufbewahrt. Das Filtrat und die Waschphasen wurden vereinigt, durchgeschüttelt und die organische Phase abgetrennt. Die wäßrige Phase wurde noch dreimal mit $CH_2Cl_2$ extrahiert. Die vereinigten organischen Phasen wurden über Natriumsulfat getrocknet, rotierend i.V. eingeengt und destilliert. Es wurden 78 g R(-)Glycidylbutyrat erhalten (0,55 Mol, 80 % Ausbeute, bezogen auf die bei 55 % Hydrolyse theoretisch erhältlichen 0,67 Mol R(-)Glycidylbutyrat). Drehwert $[\alpha]^{D}20$ = 30,00, Kp 83° C/14 mbar.

Das Immobilisat konnte mehrfach wiederverwendet werden, wobei die Aktivität langsam nachließ und sich bei ca. 70 % weitgehend stabilisierte.

**Ansprüche**

1. Verfahren zur Herstellung eines Biokatalysators durch Mischen eines Trägers mit einer Lipase und einer wäßrigen Pufferlösung bei pH 5 bis 9 und 0 bis 40° C, nach 1 Minute bis 3 Tagen Filtrieren und Waschen des Filterkuchens, dadurch gekennzeichnet, daß

a) als Träger hydrophilierte Polyolefinfasern und

b) als Lipase rohe Schweinepankreaslipase eingesetzt werden, und

c) der wäßrigen Mischung mindestens ein wasserlöslicher 2- bis 6-wertiger Alkohol mit 2 bis 6 Kohlenstoffatomen zugemischt wird.

2. Verfahren nach Anspruch 1, dadurch gekennzeichnet, daß als Träger hydrophilierte Polyethylenfasern verwendet werden.

3. Verfahren nach einem der Ansprüche 1 bis 3, dadurch gekennzeichnet, daß als wasserlöslicher Alkohol mindestens ein mehrwertiger Alkohol oder eine Mischung ein- und mehrwertiger Alkohole eingesetzt wird.

4. Verfahren nach Anspruch 3, dadurch gekennzeichnet, daß als wasserlöslicher Alkohol Glyzerin oder eine Mischung aus Glyzerin und mindestens einer Komponente aus der Gruppe bestehend aus Methanol, Ethanol, Isopropanol und 1,5-Pentandiol eingesetzt wird.

5. Verfahren nach Anspruch 3, dadurch gekennzeichnet, daß als wasserlöslicher Alkohol eine Mischung aus Glyzerin und Isopropanol eingesetzt wird.

6. Verfahren nach einem der Ansprüche 1 bis 5, dadurch gekennzeichnet, daß der Filterkuchen mit einem Vernetzungsmittel behandelt wird.

7. Verfahren nach Anspruch 6, dadurch gekennzeichnet, daß als Vernetzungsmittel Glutardialdehyd eingesetzt wird.

8. Verfahren nach Anspruch 6, dadurch gekennzeichnet, daß als Vernetzungsmittel ein wasserlöslicher Glycidylether eingesetzt wird, der hergestellt ist durch Anlagerung von 1 bis 30 Äquivalenten eines Epoxids der Formel I

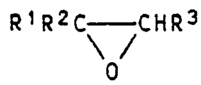

I,

worin die Reste $R^1$, $R^2$ und $R^3$ Wasserstoffatome oder Methyl- oder Ethylgruppen darstellen, pro Äquivalent (bezogen auf OH-Gruppen) eines polyfunktionellen Alkohols mit 2 bis 6 Kohlenstoffatomen oder eines Mono- oder Disaccharids und Umsetzung des so erhaltenen Anlagerungsproduktes mit einem Mol Epichlorhydrin pro Äquivalent OH und anschließende Cyclisierung zum Epoxid.

9. Verfahren nach einem der Ansprüche 1 bis 8, dadurch gekennzeichnet, daß man die Komponenten in folgenden Mengenverhältnissen mischt:

a) 1 bis 50 % hydrophilierte Polyolefinfasern,
b) 1 bis 30 % rohe Schweinepankreaslipase,
c) 1 bis 68 % eines oder mehrerer der genannten wasserlöslichen Alkohole,
d) 30 bis 97 % Wasser

sowie den pH-Wert regulierende Zusätze, wobei sich die Prozentangaben jeweils auf das Gewicht der Gesamtmischung beziehen.

10. Biokatalysator, erhältlich nach einem der Ansprüche 1 bis 9.

11. Verwendung des Biokatalysators nach Anspruch 10 zur Racematspaltung von Estern razemischer Alkohole in wasserhaltigem Medium.

12. Verwendung des Biokatalysators nach Anspruch 10 zur Razematspaltung von Glycidylbutyrat.